(19) Europäisches Patentamt

**European Patent Office**

**Office européen des brevets**

(11) Numéro de publication: **0 306 389 B1**

(12) **FASCICULE DE BREVET EUROPEEN**

(45) Date de publication de fascicule du brevet: **08.07.92**

(21) Numéro de dépôt: **88402116.3**

(22) Date de dépôt: **17.08.88**

(51) Int. Cl.⁵: **A61F 6/04**, A61B 19/04, B29C 41/22, B32B 25/02, A61L 31/00

(54) **Application de microcapsules à la fabrication d'un dispositif prophylactique en matériau élastomère, comme un préservatif ou analogue et son procédé de fabrication.**

(30) Priorité: **20.08.87 FR 8711753**

(43) Date de publication de la demande:
**08.03.89 Bulletin 89/10**

(45) Mention de la délivrance du brevet:
**08.07.92 Bulletin 92/28**

(84) Etats contractants désignés:
**AT BE CH DE ES FR GB GR IT LI LU NL SE**

(56) Documents cités:
**EP-A- 0 079 015          EP-A- 0 089 780**
**EP-A- 0 126 537          BE-A- 681 821**
**FR-A- 1 450 673          FR-A- 1 562 800**
**FR-A- 2 344 399          US-A- 2 586 674**
**US-A- 3 633 216          US-A- 4 332 243**
**US-A- 4 385 024**

(73) Titulaire: **HUTCHINSON**
**2 rue Balzac**
**F-75008 Paris(FR)**

(72) Inventeur: **Busnel, René Guy**
**Chemin de la Butte du Diable**
**F-91750 Bièvres(FR)**
Inventeur: **Argy, Gilles**
**15ter, rue Nationale**
**F-78940 La Oueue les Yvelines(FR)**

(74) Mandataire: **Orès, Bernard et al**
**Cabinet ORES 6, Avenue de Messine**
**F-75008 Paris(FR)**

Rank Xerox (UK) Business Services

## Description

L'invention a pour objet un dispositif prophylactique en matériau élastomère et son procédé de fabrication.

Elle vise, en particulier, mais sans que cette indication ait quelque caractère limitatif que ce soit, des préservatifs propres à être utilisés en tant qu'obstacle à la contamination de leurs utilisateurs par des germes ou virus lors de rapports sexuels, mais également des produits ayant un effet de protection analogue lors de la pratique de certains examens médicaux, comme des doigtiers, ou encore lors de l'exécution de certaines interventions de chirurgie ou de l'art dentaire, pour lesquelles les médecins ou dentistes utilisent de gants de protection.

Aussi bien dans les cas d'examen ou d'intervention que pour la protection à l'encontre d'agents des maladies sexuellement transmissibles, une rupture ou même parfois simplement une fissure de la membrane généralement caoutchoutique qui constitue le préservatif, le doigtier ou les gants peut entraîner une contamination du porteur du dispositif, dont l'utilisation n'est ainsi pas sans risque. Ce dernier qui est mesuré par la valeur du facteur NQA (Niveau de Qualité Acceptable) est actuellement de l'ordre de 0,4 % pour des préservatifs c'est-à-dire une valeur jugée non satisfaisante par l'ensemble du corps médical. Pour tenter de réduire la marge de risque, on a déjà proposé d'associer à des preservatifs un produit actif contre le virus du SIDA appliqué en tant que revêtement de la gaine de caoutchouc formant le préservatif. Si un tel dispositif est théoriquement satisfaisant, il peut néanmoins présenter des inconvénients dûs par exemple à d'éventuelles interactions du produit actif et du polymère de la gaine susceptibles d'entraîner une modification des propriétés élastiques et de vieillissement de cette dernière ou encore, et inversement, une perte d'activité des molécules du produit actif par adsorption et formation de complexe sur le caoutchouc et d'autres polymères. En outre la présence d'un produit actif rapporté sous forme d'un film sur l'une ou les deux faces de la gaine de caoutchouc, d'où il est transféré sur la peau et/ou les muqueuses des partenaires sexuels peut amener, en cas d'utilisation répétée, à des processus d'attaque ou d'irritation des muqueuses, ou d'allergie, ou d'hypersensibilité, etc...

On connaît depuis un certain temps (cf.notamment le Brevet US 2 586 674) des dispositifs formés d'une association de deux gaines non solidarisées entre lesquelles se situe un liquide à activité pharmacologique : outre le fait que les principes actifs mis en contact direct avec le latex ou ses dérivés agissent sur ces derniers en détruisant, et souvent très rapidement, les propriétés mécaniques et donc, par conséquent, l'imperméabilité du latex, il est également à noter que le liquide contenu entre les deux gaines a tendance, au moment du frottement, à se rassembler en un seul endroit, laissant ainsi toute une surface de l'organe à protéger sans protection.

C'est, par conséquent, un but général de l'invention de fournir un dispositif prophylactique en matériau élastomère qui pallie les inconvénients mentionnés ci-dessus.

C'est aussi un but de l'invention de fournir un procédé de fabrication d'un tel dispositif prophylactique.

Ces buts sont atteints, et d'autres encore, dans un dispositif prophylactique en matériau élastomère, du type comprenant au moins deux couches (11, 12) en matériau élastomère disposées l'une sur l'autre avec interposition entre les deux dites couches d'au moins un produit ou une substance active à l'encontre des germes, (virus, champignons, et autres agents pathogènes), ou des spermatozoïdes, ladite ou lesdites substances actives étant enfermées dans des microcapsules, qui se caractérise en ce que les microcapsules ont une dimension moyenne en diamètre de l'ordre de 5 à 50 $\mu$m et que les couches de matériau élastomère ont une épaisseur de 10 à 50 $\mu$ dans le cas de préservatifs ou une épaisseur de l'ordre de 300 $\mu$ à 500$\mu$ dans le cas de doigtiers ou de gants, de sorte que la paroi des microcapsules se rompe soit sous l'action des forces de frottement ou de cisaillement qui leur sont appliquées lorsque le dispositif est mis en place sur l'organe à protéger, soit sous l'action des microdéchirures pouvant entraîner la perméabilité du dispositif prophylactique.

Les microcapsules obtenues par le procédé de coacervation ou par d'autres méthodes classiques sont connues dans la technique d'inclusion de produits pharmaceutiques ainsi que par exemple dans des papiers autocopiants, ou des substrats souples analogues.

Une telle structure du dispositif ne libère le produit actif au contact de la peau et/ou des muqueuses de son utilisateur et/ou des partenaires sexuels qu'en cas de rupture de l'une des couches d'élastomère qui le constituent, de sorte que les effets négatifs d'accoutumance ne sont pas à craindre, d'une part, et tout phénomène d'interaction entre le caoutchouc ou tout autre matériau élastomère constitutif des couches et le produit actif est évité, d'autre part. On peut alors choisir comme produit actif une ou un mélange de plusieurs molécules compatibles chimiquement ayant l'effet pharmacologique recherché, antiviral, par exemple agissant sur le virus du SIDA, mais aussi de l'herpès, etc...ou spermicide, ou fongicide, ou trichomonacide, ou bactéricide, ou global.

Le procédé selon l'invention de fabrication d'un dispositif prophylactique tel que défini ci-dessus est alors caractérisé en ce que l'on façonne une première couche de matériau élastomère à la forme voulue du dispositif prophylactique par les techniques usuelles de cette fabrication, en ce que l'on effectue sur ladite première couche un premier traitement de pré-vulcanisation, en ce que l'on rapporte sur l'une des faces de ladite couche un film ou pellicule forme de microcapsules contenant un ou des produits actifs, en ce que l'on dépose ensuite sur ledit film ou pellicule une deuxième couche d'élastomère ayant la forme du dispositif recherché et en ce qu'on vulcanise l'ensemble qui est ensuite retiré de la forme.

Dans une réalisation préférée, les microcapsules sont déposées sur la première couche d'élastomère à l'aide d'un lit fluidisé, ou analogue, dans lequel est placé ladite première couche disposée sur sa forme et à l'état pré-vulcanisé, c'est-à-dire dans une condition telle que les microcapsules adhèrent à ladite couche dès contact avec celle-ci, par effet de "tack".

Dans une autre réalisation, une forme de fabrication sur laquelle a été déposée une première couche d'élastomère, et qui a subi un traitement de pré-vulcanisation, est immergée dans un latex qui contient, outre l'élastomère en dispersion, également les microcapsules renfermant le ou les produits actifs, cette phase étant suivie du dépôt sur la couche intermédiaire comportant les microcapsules, d'une nouvelle couche d'élastomère, puis d'un traitement de vulcanisation de l'ensemble.

Le procédé de fabrication d'un dispositif prophylactique tel que décrit ci-dessus, et qui est celui d'un dispositif à deux couches d'élastomère renfermant entre elles un film ou feuille de microcapsules contenant un ou des produits actifs, -déposé suivant le premier ou le second mode d'exécution-, peut bien entendu être répété pour la fabrication de dispositifs à un plus grand nombre de couches, et qui ainsi, non seulement présentent une solidité mécanique et une imperméabilité accrues mais également permettent d'associer au dispositif une plus grande quantité de produit(s) actif(s).

Un tel procédé qui rend mécaniquement solidaire la première et seconde enveloppes en raison de la présence de "ponts" ou "pontets" d'élastomère entre les deux couches constitutives de chacune des enveloppes est favorable au comportement mécanique d'ensemble du dispositif, la mise en place de celui-ci sur le pénis ou sur le doigt ou la main du praticien pratiquant un examen ou une intervention chirurgicale développant des forces suffisantes pour provoquer la cassure, rupture ou éclatement des gaines des microcapsules et ainsi la libération du produit actif dans l'espace ménagé entre les couches d'élastomère voisines.

Selon un mode de réalisation avantageux de l'objet de l'invention, les microcapsules renfermant les principes actifs ont une dimension moyenne en diamètre de l'ordre de 5 à 50 $\mu$, les couches en matériau élastomère ayant une épaisseur de 10 à 50 $\mu$ dans le cas de préservatifs ou une épaisseur plus grande, pouvant atteindre 300 à 500 $\mu$, dans le cas de doigtiers ou de gants.

Ces dimensions ont été choisies de manière à ce qu'une dynamique de faible amplitude (comportant deux à trois mouvements du sexe masculin porteur du préservatif et pour les gants une pression de 300 à 500 g identique à celle exercée lors d'un essuyage par exemple) provoque d'une part l'éclatement des microcapsules, l'épandage de l'agent pharmacologique et, d'autre part, grâce à la déchirure de la fine couche intérieure de latex produite en même temps, le recouvrement de l'organe porteur (sexe, doigt ou analogue).

D'autres caractéristiques et avantages de l'invention apparaîtront de la description qui suit, faite à titre d'exemple et en référence au dessin annexé dans lequel :

- la Figure 1 est une vue schématique, à grande échelle, et en coupe illustrant la structure et un premier mode d'exécution d'un procédé de fabrication selon l'invention ;
- la Figure 2 est une vue analogue à la Figure 1 pour un second mode d'exécution ;
- la Figure 3 est une vue très schématique d'une partie du dispositif prophylactique selon l'invention.

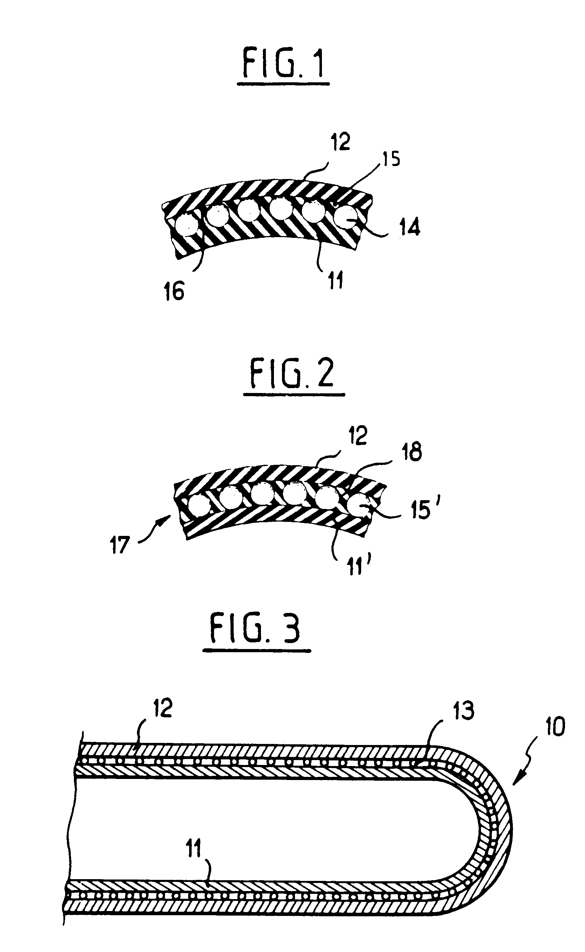

Comme montré sur cette dernière Figure, le dispositif prophylactique comme un préservatif, un doigtier, des gants de chirurgie ou analogue 10 comprend essentiellement deux couches 11 et 12 en élastomère, par exemple à base de caoutchouc naturel entre lesquelles est emprisonné un ou des produits actifs 13 sous forme de microcapsules, microgranules ou analogues. A cet égard, la représentation de la Figure 3 est très schématique et ne doit pas être considérée comme donnant une indication d'échelle des parties constitutives du dispositif. En fait, les microcapsules renfermant le ou les produits actifs ont une dimension moyenne en diamètre de l'ordre de 5 à 50 $\mu$, les couches de matériau élastomère ayant quant à elles une épaisseur de 10 à 30 $\mu$ dans le cas de préservatifs ou une épaisseur plus grande, pouvant atteindre 300 $\mu$ dans le cas de doigtiers ou de gants.

Les microcapsules ou microsphérules constituent un système matriciel dont la coque est en un matériau connu, comme par exemple : acétophtalate de cellulose, alcool polyvinylique, pectine, gomme arabique, méthylcellulose, gélatine, résine époxy, ou analogues, renfermant un ou plusieurs produits ayant des propriétés spécifiques et complémentaires désirées notamment des propriétés antivirales, trichomonacides, fongicides, germicides, spermatocides, lesdits produits étant pris dans le groupe qui comprend notamment d'une manière non limitative : le chlorydrate de moroxydine, la vidarabine, l'aciclovir, l'iodo-5-desoxy-cytidine, l'idoxyridine (D.C.I.) ; -les ammoniums quaternaires, le chlorure de diméthyl-alkylbenzyl ammonium ou le chlorure de benzalkonium , l'hexyl resorcinol avec pH acide, par ajout de bromure de benzyldodécinium ou un pH neutre, avec lauryl-sulfate de sodium, le nonoxynol, le paradi-isobutylphénoxypolyéthoxyéthanol, le benséthonium chlorure (D.C.I.) le nitrate phényl-mercurique avec addition de parahydroxybenzoate de méthyle ; -le nitrate de miconazole, le nitrate d'éconazole, la nystatine, le nifuratel, la natamycine ; l'Acétarsol, le Chlorquinaldol (5,7-dichloro-8-hydroxyquinaldine), le Ténonitrozole (D.C.I.) , le Ternonidazole (D.C.I.) ; les polyvinylpyrrolidones iodés, la chlorhexidine (D.C.I.) digluconate, le sulfate de néomycine (D.C.I.) le sulfate de polymyxine B (D.C.I.) ; l'hypochloride de sodium, le permenganate de potassium, les nitrates d'argent, les dérivés mercuriques ; -les molécules actives étant le cas échéant complétées par l'ajout d'excipient et/ou de conserveur.

Pour la fabrication d'un dispositif selon l'invention, on prévoit de déposer sur une forme en céramique, en verre ou en un matériau analogue définissant le gant, le doigtier, ou le préservatif à fabriquer une première couche 12 d'élastomère ; celle-ci peut être obtenue par immersion dans un latex avec fixation capillaire sous l'action d'agents coagulants ou avantageusement à l'aide d'un procédé d'électrophorèse. La forme revêtue de ladite première couche est alors soumise à un traitement de pré-vulcanisation, dans un tunnel à air chaud, la température de celui-ci et le temps de passage étant réglés pour que l'élastomère ne soit pas totalement vulcanisé et conserve ainsi des propriétés adhérentes mises à profit lorsque, à la sortie dudit tunnel, la forme revêtue de sa couche d'élastomère pré-vulcanisée est plongée dans un lit fluidisé de microcapsules 14 contenant le ou les principes actifs. Celles-ci se déposent au contact de la face externe 15 de la couche 12 et restent fixées par adhérence à ladite couche. L'ensemble est alors revêtu de la couche 11, avantageusement par plongée dans un bain de latex, l'élastomère de ladite couche enrobant les microcapsules 14 et les espaces laissés libres entre lesdites microcapsules pour former des ponts ou pontets comme ceux montrés en 16 sur la Figure 1 qui assurent la solidarisation mécanique des couches 11 et 12 en même temps que l'emprisonnement des microcapsules 14.

On procède ensuite à la vulcanisation de l'ensemble ainsi préparé par un second passage dans un tunnel à air chaud dont la température est, en tout état de cause, inférieure à celle susceptible de détériorer le ou les produits actifs et/ou la coque des microcapsules ou microsphérules ainsi noyées entre les deux couches d'élastomère.

On peut avantageusement utiliser des solutés d'un ou de plusieurs produits actifs ayant des températures de vaporisation supérieures à la température habituelle de vulcanisation des élastomères, tels que l'huile de silicone, l'éthylèneglycol ou tout autre solvant compatible sur le plan chimique et pharmacologique. Pour les substances hydrosolubles, il peut être très avantageux de polymériser l'élastomère dans une étuve sous pression, ce qui évitera l'éclatement des microgélules sous l'effet de la tension de vapeur.

Dans un second mode d'exécution, illustré sur la Figure 2, la première couche 12 est fabriquée comme indiqué ci-dessus jusqu'à la phase de prévulcanisation. Cette phase est ensuite suivie d'une immersion non plus dans un lit fluidisé de microcapsules mais dans un latex dans lequel ont été dispersées de façon homogène les microcapsules 15', de sorte que l'on forme sur la couche 12 une couche intermédiaire 17 renfermant les microcapsules 15' dans une matrice d'élastomère 18 qui assure la liaison mécanique entre la couche 12 et la couche 11', - analogue à la couche 11-, laquelle est rapportée sur la matrice 18 et les microcapsules 15' par trempage dans un latex de façon analogue à ce qui a été décrit ci-dessus. Après vulcanisation de l'ensemble ainsi préparé, on obtient le dispositif prophylactique recherché.

Bien que l'on ait décrit, en référence aux Figures 1 et 2, un dispositif à deux couches d'élastomère renfermant entre elles un ou des produits actifs sous forme de micro-capsules, il va de soi que les opérations indiquées peuvent être répétées pour former un dispositif multi-couches, ayant ainsi une solidité mécanique accrue et propre à libérer une plus grande quantité de produit(s) actif(s) en cas de nécessité.

**Revendications**

1. Dispositif prophylactique en matériau élastomère comme un préservatif, un doigtier, un gant ou analogue comprenant au moins deux couches (11, 12) en matériau élastomère disposées l'une sur l'autre avec interposition entre les deux dites couches d'au moins un produit ou une substance active à l'encontre des germes, (virus, champignons, et autres agents pathogènes), ou des spermatozoïdes, ladite ou lesdites substances actives étant enfermées dans des microcapsules, caractérisé en ce que les microcapsules ont une dimension moyenne en diamètre de l'ordre de 5 à 50 $\mu$m et que les couches de matériau élastomère ont une épaisseur de 10 à 50 $\mu$ dans le cas de préservatifs ou une épaisseur de l'ordre de 300 $\mu$ à 500$\mu$ dans le cas de doigtiers ou de gants, de sorte que la paroi des microcapsules se rompe soit sous l'action des forces de frottement ou de cisaillement qui leur sont appliquées lorsque le dispositif est mis en place sur l'organe à protéger, soit sous l'action des microdéchirures pouvant entraîner la perméabilité du dispositif prophylactique.

2. Dispositif selon la revendication 1, caractérisé en ce que les microcapsules ou les microsphérules renfermant un ou plusieurs produits ayant des propriétés spécifiques et complémentaires désirées, notamment des propriétés antivirales, trichomonacides, fongicides, germicides, spermatocides, lesdits produits étant pris dans le groupe qui comprend notamment le chlorhydrate de moroxydine, la vidarabine, l'aciclovir, l'iodo-5-désoxy-cytidine, l'idoxyridine (D.C.I.), les ammoniums quaternaires, le chlorure de diméthylalkyl-benzyl ammonium ou le chlorure de benzalkonium, l'hexyl résorcinol avec pH acide, par ajout de bromure de benzyldodécinium ou à pH neutre, avec laurylsulfate de sodium, le nonoxynol, le paradiisobutylphénoxypolyéthoxyéthanol, le benséthonium chlorure (D.C.I.), le nitratephényl-mercurique avec addition de parahydroxybenzoate de méthyle, le nitrate de miconazole, le nitrate d'éconazole, la nystatine, le nifuratel, la natamycine, l'acétarsol, le chlorquinaldol (5,7-dichloro-8-hydroxyquinaldine), le ténonitrozole (D.C.I.), le ternonidazole (D.C.I.), les polyvinyl-pyrrolidones iodés, la chlorhexidine (D.C.I.), le digluconate, le sulfate de néomycine (D.C.I.), le sulfate de polymyxine B (D.C.I.), l'hypochloride de sodium, le permenganate de potassium, les nitrates d'argent, les dérivés mercuriques ;
   - les molécules actives étant, le cas échéant, complémentées par l'ajout d'excipient et/ou de conserveur.

3. Procédé de fabrication d'un dispositif prophylactique selon l'une quelconque des Revendications 1 et 2, selon lequel on façonne une couche de matériau élastomère à la forme voulue du dispositif prophylactique par les techniques usuelles, caractérisé en ce que l'on effectue sur ladite première couche un premier traitement de prévulcanisation, en ce que l'on rapporte sur l'une des faces de ladite couche un film ou pellicule en forme de microcapsules contenant un ou des produits actifs, en ce que l'on dépose ensuite sur ledit film ou pellicule une deuxième couche d'élastomère ayant la forme du dispositif recherché et en ce qu'on vulcanise l'ensemble qui est ensuite retiré de la forme.

4. Procédé selon la revendication 3, caractérisé en ce que les microcapsules sont déposées sur la première couche d'élastomère à l'aide d'un lit fluidisé ou analogue dans lequel on place ladite première couche disposée sur sa forme et à l'état pré-vulcanisé, c'est-à-dire dans une condition telle que les microcapsules adhèrent à ladite couche dès contact avec celle-ci, par effet de "tack"

5. Procédé selon la revendication 3, caractérisé en ce que la forme de fabrication sur laquelle a été déposée une première couche d'élastomère, et qui a subi un traitement de pré-vulcanisation, est immergée dans un latex qui contient, outre l'élastomère en dispersion, également les microcapsules renfermant le ou les produits actifs, cette phase étant suivie du dépôt sur la couche intermédiaire comportant les microcapsules, d'une nouvelle couche d'élastomère, puis d'un traitement de vulcanisation de l'ensemble.

6. Procédé de fabrication selon l'une quelconque des revendications 3 à 5, caractérisé en ce qu'il est répété n fois pour la fabrication d'un dispositif à plus de deux couches d'élastomère, et qui ainsi, non seulement présente une solidité mécanique accrue, mais également permet d'associer au dispositif une plus grande quantité de produit(s) actif(s).

7. Procédé de fabrication selon l'une quelconque des revendications 3 à 6, caractérisé en ce que les agents chimiques à effet pharmacologique inclus dans les microcapsules ou les microsphérules, sont mis en suspension dans une solution qui entraîne, lors de la phase finale de vulcanisation de l'élastomère selon la Revendication 3, la mise en oeuvre d'un étuvage sous pression.

8. Procédé de fabrication selon la revendication 7, caractérisé en ce que la solution est une solution aqueuse.

**Claims**

1. Prophylactic device made from elastomeric material as a condom, finger-stall, glove or similar, comprising at least two layers (11, 12) of elastomeric material disposed one above the other with the interposing between the said two layers of at least one product or one substance active against germs (viruses, fungi and other pathogenic agents) or spermatozoa, the said active substance or substances being enclosed in microcapsules, characterised in that the microcapsules have a mean dimension in diameter of the order of 5 to 50 $\mu$m and in that the layers of elastomeric material have a thickness of 10 to 50 $\mu$ in the case of condoms or a thickness of the order of 300 $\mu$ to 500 $\mu$ in the case of finger-stalls or gloves, so that the walls of the microcapsules break either under the effect of the frictional or shearing forces which are applied to them when the device is placed on the organ to be protected, or under the effect of microtears which may cause permeability in the prophylactic device.

2. Device according to claim 1, characterised in that the microcapsules or microspherules containing one or more products having desired specific and complementary properties, notably anti-viral, trichomonacidal, fungicidal, germicidal or spermatocidal properties, the said products being taken from amongst the group which comprises notably moroxydine hydrochloride, vidarabine, aciclovir, iodo-5-desoxycytidine, idoxyridine (D.C.I.); - quaternary ammoniums, ammonium dimethyl alkyl benzyl chloride or benzalkonium chloride, hexyl resorcinol with an acid pH, by the addition of benzyl dodecinium bromide, or a neutral pH, with sodium lauryl sulphate, nonoxynol, para diisobutyl phenoxy-polyethoxy ethanol, bensethonium chloride (D.C.I.), phenyl mercuric nitrate with the addition of methyl parahydroxybenzoate, miconazole nitrate, econazole nitrate, nystatine, nifuratel, natamycine, acetarsol, chlorquinaldol (5,7-dichloro-8-hydroxyquinaldine), tenonitrozole (D.C.I.), ternonidazole (D.C.I.); iodine polyvinyl pyrrolidones, chlorhexidine (D.C.I.), digluconate, neomycine sulphate (D.C.I.), polymyxine B sulphate (D.C.I.); sodium hypochloride, potassium permanganate, silver nitrate and mercury derivatives;
    - the active molecules being if necessary supplemented by the addition of excipient and/or preservative.

3. Method of manufacturing a prophylactic device according to either one of claims 1 and 2, according to which a layer of elastomer material is formed in the desired shape of the prophylactic device by the normal techniques, characterised in that a first prevulcanising treatment is carried out on the said first layer, in that a film or skin in the form of microcapsules containing one or more active products is put on one of the faces of the said layer, in that a second layer of elastomer having the shape of the device sought for is then placed on the said film or skin and in that the whole is vulcanised and then withdrawn from the form.

4. Method according to claim 3, characterised in that the microcapsules are deposited on the first layer of elastomer by means of a fluidised bed or similar in which the said first layer is placed arranged on its form and in the prevulcanised state, that is to say in a condition such that the microcapsules adhere to the said layer as soon as they make contact with the latter, by "tack" effect.

5. Method according to claim 3, characterised in that the manufacturing form on which a first layer of elastomer has been placed, and which has undergone a prevulcanising treatment, is immersed in a latex which contains, in addition to elastomer in dispersion, also the microcapsules containing the active product or products, this stage being followed by the placing of a further layer of elastomer on the intermediate layer comprising the microcapsules, and then a vulcanising treatment of the whole.

6. Method of manufacture according to any one of claims 3 to 5, characterised in that it is repeated n times for the manufacture of a device with more than two layers of elastomer and which thus not only has increased mechanical strength but also makes it possible to associate a larger quantity of active product or products with the device.

7. Method of manufacture according to any one of claims 3 to 6, characterised in that the chemical agents with a pharmacological effect included in the microcapsules or microspherules are placed in suspension in a solution which entails, during the final stage of vulcanising of the elastomer according to claim 3, the implementation of stoving under pressure.

8. Method of manufacture according to claim 7, characterised in that the solution is an aqueous solution.

**Patentansprüche**

1. Prophylaktische Vorrichtung aus elastomerem Material als Präservativ, Fingerschutz, Handschuh oder Analogon davon, umfassend mindestens zwei Schichten (11, 12) aus elastomerem Material, die aufeinander angebracht sind, wobei zwischen den beiden Schichten mindestens ein Produkt oder eine aktive Substanz gegen Keime (Viren, Pilze und andere pathogene Erreger) oder Spermien vorhanden ist, wobei die aktive Substanz bzw. die aktiven Substanzen in Mikrokapseln eingeschlossen sind, **dadurch gekennzeichnet,** -daß die Mikrokapseln einen durchschnittlichen Durchmesser in der Größenordnung von 5 bis 50 $\mu m$ besitzen und daß die Schichten aus elastomerem Material eine Dicke von 10 bis 50 $\mu$ im Fall von Präservativen oder eine Dicke in der Größenordnung von 300 $\mu$ bis 500 $\mu$ im Fall von Fingerschutz oder Handschuhen besitzen, so daß die Umhüllung der Mikrokapseln entweder unter Einwirkung von Reibungs- oder Scherkräften, die auf ihnen entstehen, wenn die Vorrichtung auf ein zu schützendes Organ aufgebracht wird oder unter Wirkung von Mikrorissen, die die Permeabilität der prophylaktischen Vorrichtung zur Folge haben können, aufbricht.

2. Vorrichtung nach Anspruch 1, **dadurch gekennzeichnet,** daß die Mikrokapseln oder die Mikrokörperchen ein oder mehrere Produkt/Produkte mit erwünschten spezifischen und komplementären Eigenschaften, insbesondere mit antiviralen, trichomonaziden, fungiziden, germiziden, spermatoziden Eigenschaften umhüllen, wobei die Produkte aus der Gruppe, umfassend insbesondere Moroxydinchlorhydrat, Vidarabin, Aciclovir, 5-Ioddesoxycytidin, Iodoxyridin (D.C.I.), quarternäre Ammoniumverbindungen, Dimethylalkylbenzylammoniumchlorid oder Benzalkoniumchlorid, Hexylresorcinol mit saurem pH durch Zugabe von Benzyldodeciniumbromid oder neutralem pH mit Natriumlaurylsulfat, Nonoxynol, Paradiisobutylphenoxypolyethoxyethanol, Bensethoniumchlorid (D.C.I.), Phenylquecksilbernitrat durch Zugabe von Methylparahydroxybenzoat, Miconazolnitrat, Econazolnitrat, Nystatin, Nifuratel, Natamycin, Acetarsol, Chlorchinaldol (5,7-Dichlor-8-hydroxychinaldin), Tenonitrozol (D.C.I.), Ternonidazol (D.C.I.), Polyvinylpyrrolidoniodid, Chlorhexidin (D.C.I.), Digluconat, Neomycinsulfat (D.C.I.), Polymyxin-B-sulfat (D.C.I.), Natriumhypochlorit, Kaliumpermanganat, Silbernitrate, Quecksilberderivate, ausgewählt ist, wobei die Wirkstoffe ggf. durch Zusatz eines Excipiens und/oder Konservierungsstoffs ergänzt sind.

3. Verfahren zur Herstellung einer prophylaktischen Vorrichtung nach einem der Ansprüche 1 und 2, wobei man eine Schicht aus elastomerem Material mit der gewünschten Form der prophylaktischen Vorrichtung nach an sich bekannten Techniken herstellt, **dadurch gekennzeichnet,** daß man auf der ersten Schicht eine erste Vorvulkanisationsbehandlung durchführt, auf eine der Oberflächen der Schicht einen Film oder ein Häutchen in Form von Mikrokapseln, welche einen oder mehrere Wirkstoff/Wirkstoffe enthalten, aufbringt, anschließend auf dem Film oder dem Häutchen eine zweite Elastomerenschicht mit der Form der gewünschten Vorrichtung aufbringt und das Ganze vulkanisiert und anschließend von der Form zieht.

4. Verfahren nach Anspruch 3, **dadurch gekennzeichnet,** daß man die Mikrokapseln auf der ersten Elastomerenschicht mit Hilfe eines fluidisierten Bettes oder Analogons aufbringt, in welches man die erste Schicht in ihrer Form und in vorvulkanisiertem Zustand einbringt, d.h. in einem Zustand, in dem die Mikrokapseln an der Schicht gleich nach Kontakt damit durch den "Tack"- bzw. Klebeeffekt haften.

5. Verfahren nach Anspruch 3, **dadurch gekennzeichnet,** daß die Form zur Herstellung, auf welcher eine erste Elastomerenschicht aufgebracht wurde und die einer Vorvulkanisationsbehandlung unterzogen wurde, in einen Latex eingetaucht wird, der außer dem Elastomeren in Dispersion ebenso Mikrokapseln enthält, die das oder die Wirkstoff/Wirkstoffe umhüllen, wobei sich an diese Phase die Abscheidung einer neuen Elastomerenschicht auf der die Mikrokapseln tragenden Zwischenschicht und anschließend eine Vulkanisationsbehandlung des Ganzen anschließen.

6. Verfahren zur Herstellung nach einem der Ansprüche 3 bis 5, **dadurch gekennzeichnet,** daß es n-mal zur Herstellung einer Vorrichtung mit mehr als zwei elastomeren Schichten und die nicht nur eine erhöhte mechanische Festigkeit zeigt, sondern ebenso die Assoziation einer größeren Menge an Wirkstoff bzw. Wirkstoffen an die Vorrichtung gestattet, wiederholt wird.

7. Verfahren zur Herstellung nach einem der Ansprüche 3 bis 6, **dadurch gekennzeichnet,** daß die chemischen Mittel mit pharmakologischer Wirkung, die in den Mikrokapseln oder den Mikrokörperchen eingeschlossen sind, in einer Lösung suspendiert werden, die in der Endphase der Vulkanisation des Elastomeren nach Anspruch 3 die Verwendung eines Trockenofens unter Druck zur Folge hat.

8. Verfahren zur Herstellung nach Anspruch 7, **dadurch gekennzeichnet,** daß die Lösung eine wäßrige Lösung ist.

FIG. 1

FIG. 2

FIG. 3